# FASCICULE DE BREVET EUROPEEN

(11) **EP 3 233 003 B1**
(45) Date de publication et mention de la délivrance du brevet: **26.09.2018**
(21) Numéro de dépôt: 15823686.9
(22) Date de dépôt: 18.12.2015
(51) Int. Cl.: A61F 13/00, A61L 15/22, A61L 15/34, A61L 15/42, A61L 15/44

(54) **PANSEMENT COMPRENANT UN SUPPORT ET UNE MATRICE ELASTOMERIQUE HYDROPHOBE**
WUNDVERBAND MIT EINEM TRÄGER UND EINER HYDROPHOBEN ELASTOMEREN MATRIX
DRESSING COMPRISING A SUPPORT AND A HYDROPHOBIC ELASTOMERIC MATRIX

(30) Priorité: 19.12.2014 FR 1463029
(43) Date de publication de la demande: 25.10.2017
(73) Titulaire: URGO RECHERCHE INNOVATION ET DEVELOPPEMENT, 21300 Chenôve (FR)
(72) Inventeur: PASQUINET, Laurent, 21800 Crimolois (FR); DESMAISON, Nadège, 21110 Tart le Haut (FR); DANEROL, Anne-Sophie, 21000 Dijon (FR)
(74) Mandataire: Hubert, Philippe
(86) Numéro de dépôt international: PCT/FR2015/053647
(87) Numéro de publication internationale: WO 2016/097653

(56) Documents cités:
- EP-A1- 2 524 706
- EP-B1- 1 143 895
- WO-A1-2012/131263
- WO-A1-2013/093213
- US-A- 6 017 854

## Description

La présente invention vise un nouveau pansement comprenant un support et une matrice élastomérique hydrophobe non absorbante enrobant ledit support, ladite matrice étant formée notamment par l'association d'au moins deux élastomères de type SEPS, SEBS ou SEEPS, constitués d'une association de blocs polystyrène et de blocs polyoléfine, chacun de ces élastomères ayant un poids moléculaire respectif compris entre 200000 et 350000 Dalton et entre 320000 et 600000 Dalton mesurés par chromatographie par perméation de gel, la part totale d'élastomère étant comprise entre 3,2 et 10% du poids total de ladite matrice.

L'invention concerne un pansement comportant un support et une matrice de polymère élastomère comme couche de contact à la plaie.

Les pansements contenant des substances grasses sont utilisés depuis des années avec succès pour le traitement local de plaies traumatiques telles que des coupures et des écorchures et les plaies chroniques telles que les escarres, les ulcères variqueux, et les brûlures chez l'Etre Humain et l'animal.

Un tulle imprégné d'une matière grasse Lomatuell® H, de la société Lohmann & Rauscher constitue l'un des produits utilisés pour le soin médical des plaies cutanées. Ce produit, qui est formé d'un tissu en coton à grandes mailles imprégné d'un corps gras à base de vaseline, présente cependant des inconvénients tels que par exemple, une perte des corps gras sur les instruments de manipulation (par exemple, les gants) et sur la plaie et l'environnement de la plaie. Lors du contact avec la plaie, la vaseline se ramollit en raison de l'augmentation de température et a tendance à être poussée à la périphérie du pansement.

Ce phénomène entraîne fréquemment la mise à nu du support et le contact direct avec la plaie. L'adhérence du support à la plaie entraîne souvent des changements de pansement traumatiques, douloureux, et une gêne dans le processus de cicatrisation.

D'autres produits se trouvant dans le commerce, par exemple le pansement de la société Johnson et Johnson ADAPTIC®, qui est un tricot de viscose imbibé d'une émulsion huile dans eau ou le produit JELONET® (Société Smith et Nephew) qui est une gaze de coton imbibée de paraffine, répondent au même objectif que le Lomatuell® H mais présentent cependant également un comportement défavorable au regard de la perte des substances grasses.

On sait selon l'état actuel des connaissances, que l'addition d'une petite quantité de substances hydrophiles du groupe des hydrocolloïdes, tels que par exemple la carboxyméthylcellulose de sodium, en dispersion dans une matrice élastomère hydrophobe augmente partiellement l'hydrophilie de la matrice après contact avec l'exsudat de la plaie en formant un gel sur la surface. Ce mélange des propriétés hydrophobes et hydrophiles de la surface de la matrice qui entre en contact direct avec la plaie entraîne un résultat extrêmement favorable pour le processus de cicatrisation de la plaie : un degré d'humidité optimal maintenu en surface et la présence de corps gras qui isolent la structure du pansement, entraînent une cicatrisation plus rapide de la plaie et une absence totale d'adhérence de la compresse sur la plaie.

WO2013/093213 divulgue un pansement interface adhérent comprenant un gel cohésif non-adhérent formé d'une matrice élastomérique hydrophobe constituée d'un élastomère tribloc du type SEBS, SEPS, éventuellement associé à un copolymère dibloc, ledit élastomère étant fortement plastifié au moyen d'une huile minérale et contenant en dispersion une faible quantité d'un hydrocolloïde. Le brevet de Lohmann & Rauscher EP 2 524 706 décrit un pansement avec un support et une matrice élastomérique hydrophobe non absorbante destinée à entrer en contact avec la plaie. Cette matrice présente une part totale d'élastomère par rapport au poids total de la matrice inférieure à 3,2%, de préférence de 2,6%. Néanmoins, un tel produit souffre de certaines limites. En effet, ce produit et plus précisément sa matrice élastomérique libère une quantité importante d'huile rendant celui-ci n'étant pas parfaitement adapté à une application efficiente sur la plaie du patient. On assiste donc à une exsudation partielle de la matrice élastomérique enrobant son support.

En parallèle, le brevet des Laboratoires Urgo EP 1 143 895 décrit une compresse non adhérente stérile comprenant une matrice élastomérique hydrophobe plastifiée constituée de 100 parts en poids d'un élastomère tribloc, 1000 à 2000 parts en poids d'huile plastifiante, de 0 à 400 parts en poids de vaseline et 3 à 20% de particules hydrophiles en dispersion. Ledit produit présente de bonnes propriétés structurelles et de cicatrisation mais libère aussi un peu d'huile sur le long terme. Il serait donc souhaitable de disposer d'un produit libérant le moins d'huile possible.

En effet, jusqu'à présent il n'a jamais été mis en oeuvre de pansement interface, c'est-à-dire de pansement comprenant un support enrobé par une matrice élastomérique hydrophobe dont les niveaux de libération d'huile seraient plus faibles que les pansements interface commercialisés et connus actuellement.

Un produit présentant ces propriétés-ci serait profitable quant à l'amélioration de sa stabilité au conditionnement, notamment par le fait que sa structure serait plus stable quelques soient les conditions de températures de stockage ou usuelles auxquelles il serait soumis, ainsi que dans son usage, le personnel soignant n'ayant plus à se concentrer sur une manipulation optimale à la pose d'un tel pansement afin d'éviter toute exsudation néfaste éventuelle, préalablement à son apposition sur le lit de la plaie du patient.

Ainsi l'objet de la présente invention réside dans un pansement comprenant un support et une matrice élastomérique hydrophobe enrobant ledit support, ladite matrice étant formée notamment par l'association d'au moins deux élastomères de type SEPS, SEBS ou SEEPS, constitués d'une association de blocs polystyrène et de blocs polyoléfine, chacun de ces élastomères ayant un poids moléculaire respectif compris entre 200000 et 350000 Dalton et entre 320000 et 600000 Dalton mesurés par chromatographie par perméation de gel, la part totale d'élastomère étant comprise entre 3,2 et 10% du poids total de ladite matrice.

### Matrice élastomérique

Pour obtenir une matrice élastomérique hydrophobe selon l'objet de l'invention, on utilise de préférence des élastomères tribloc constitués d'une association de blocs polystyrène et de blocs polyoléfine de type polystyrène-b-poly(éthylène-butylène)-b-polystyrène (S-EB-S), ou polystyrène-b-poly(éthylène-propylène)-b-polystyrène (S-EP-S), ou polystyrène-b-poly(éthylène-éthylène/propylène)-b-polystyrène (S-EEP-S) de poids moléculaire élevé et ultra-élevé.

Alternativement, l'un de ces élastomères triblocs peut être remplacé par un élastomère dibloc également constitué d'une association de blocs polystyrène et de blocs polyoléfine, sous réserve que cet élastomère présente des propriétés similaires, en terme de poids moléculaire à l'élastomère tribloc auquel il a été substitué.

De manière avantageuse, on utilise des élastomères hydrogénés polystyrène-b-poly(éthylène-butylène)-b-polystyrène (S-EB-S, par exemple le Kraton® G 1651 EU). Il peut être également utilisé des élastomères hydrogénés polystyrène-b-poly(éthylène-éthylène/ propylène)-b-polystyrène (S-EEP-S, tels que les Septon® 4055, 4077 ou 4099).

Pour obtenir une matrice élastomérique hydrophobe selon ce mode de réalisation, on choisit de préférence un mélange d'au moins deux élastomères tribloc de type S-EB-S ou S-EP-S, en particulier de type S-EEP-S, l'un des deux élastomères présentant un poids moléculaire supérieur ou égal à 200 000 Dalton , de préférence supérieur ou égal à 230 000 Dalton et inférieur ou égal à 350 000 Dalton tel que mesuré par chromatographie par perméation de gel, et l'autre élastomère présentant un poids moléculaire supérieur ou égal à 320 000 Dalton et inférieur ou égal à 450 000 Dalton tel que mesuré par chromatographie par perméation de gel.

Selon un mode de réalisation préféré, on choisit un mélange d'au moins deux élastomères tribloc de type S-EB-S ou S-EP-S, en particulier de type S-EEP-S, l'un des deux élastomères présentant un poids moléculaire supérieur ou égal à 240 000 Dalton et inférieur ou égal à 310 000 Dalton tel que mesuré par chromatographie par perméation de gel, et l'autre élastomère présentant un poids moléculaire supérieur ou égal à 320 000 Dalton et inférieur ou égal à 400 000 Dalton tel que mesuré par chromatographie par perméation de gel.

De manière encore plus avantageuse, chaque élastomère possède une viscosité Brookfield d'au moins 5000 mPas (en solution à 10 % en poids dans le toluène à 30°C) .

Dans le cadre de la présente description, le poids moléculaire désigne le poids moléculaire moyen en poids.

On détermine le poids moléculaire moyen en poids de chaque élastomère (Mw) selon la même méthode que celle exposée dans le brevet EP 0 640 115. Ainsi cette détermination se fait par chromatographie par perméation de gel (GPC) dans les conditions suivantes :
- Solvant : THF pour HPLC
- Vitesse d'écoulement : 1,33 ml/min
- Température : ambiante
- Volume injecté : 200 microlitres
- Concentration de l'échantillon : 0,05%
- Standard international : phényl-hexane
- Détecteur : Détecteur différentiel d'indice de réfraction
- Colonnes : aux nombres de deux, de 60 cm de chez Polymer Labs., gel mélangé 10 microns.
- Exploitation des données : Logiciel de calibrage de Polymer labs.

On pèse soigneusement les échantillons (100 mg) et on les dissout dans 40 ml de THF dans des ballons de 50 ml. Ensuite, on ajoute 50 microlitres de marqueur quand le polymère est dissous et on complète les solutions jusqu'à la marque de 50 ml. Ensuite, on les filtre à travers un filtre sous pression de 0,2 µM et on les injecte dans la GPC. On prépare 4 solutions séparées de différents mélanges de standards de polystyrène dans des flacons de verre jaugés et on y ajoute un volume connu de marqueur.

On utilise le marqueur pour corriger les variations de flux. Le logiciel crée une courbe d'étalonnage à partir des mélanges de standards en utilisant un ajustement polynomial du troisième ordre.

Les valeurs obtenues pour différents élastomères sont répertoriées dans le tableau suivant :

| Elastomère | Poids moléculaire (Mw) |
|---|---|
| Septon® 4055 | 308 000 Dalton |
| Septon® 4077 | 392 000 Dalton |
| Septon® 4155 | 290 000 Dalton |
| Septon® 2005 | 257 000 Dalton |
| Septon® 2006 | 251 000 Dalton |
| Septon® 2105 | 275 000 Dalton |
| Septon® 2055 | 250 000 Dalton |
| Kraton® G 1651 EU | 240 000 Dalton |

Selon un mode de réalisation préféré de l'invention, la matrice hydrophobe élastomérique peut comprendre entre 3,2 et 10% en poids d'élastomères par rapport au poids total de la matrice, de préférence entre 4 et 6,5%, de façon encore plus préférée entre 4,5 et 5,5%.

Ainsi, une matrice comprenant une association d'un élastomère de la marque Kraton® tel que le Kraton® G 1651 EU ou d'un autre élastomère de la marque Kraton® de la série G possédant la propriété de poids moléculaire souhaitée, à un élastomère de la marque Septon® tel que l'élastomère Septon® 4077 ou l'élastomère Septon® 4099 est envisagée par ce mode de réalisation.

De la même manière, une matrice comprenant une association d'un élastomère de la marque Septon® tel que l'élastomère Septon® 4055 ou d'un autre élastomère de la marque Septon répertorié dans le tableau précédent ou encore d'un quelconque élastomère de la marque Septon® ayant les propriétés mentionnées précédemment et non répertorié dans le tableau précédent, à un autre élastomère de la marque Septon® tel que le Septon® 4077 ou bien l'élastomère Septon® 4099 est envisagée par ce mode de réalisation.

Selon un mode de réalisation préféré de la présente invention, la part totale au sein de la matrice hydrophobe élastomérique de l'élastomère possédant le plus haut poids moléculaire est supérieure à 5%, de préférence supérieure à 10%, tout en étant inférieure ou égale à 50% de la part totale d'élastomères introduits dans ladite matrice.

Selon un mode de réalisation encore plus préféré de la présente invention, la part totale au sein de la matrice hydrophobe élastomérique de l'élastomère possédant le plus haut poids moléculaire est supérieure à 20%, de préférence supérieure à 30%, de façon encore plus préférée supérieure à 40%, tout en étant inférieure ou égale à 50% de la part totale d'élastomères introduits dans ladite matrice.

Selon un autre mode de réalisation préféré de l'invention, la matrice élastomérique comprend au moins deux élastomères, un tribloc et un dibloc, seuls ou en mélange, ayant des propriétés en termes de poids moléculaires similaires à celles des élastomères décrits précédemment.

Outre les élastomères, la matrice hydrophobe polymérique comprend également au moins un plastifiant huileux, constituant de préférence la majorité de la matrice hydrophobe élastomérique. Le plastifiant huileux peut notamment être constitué par une huile plastifiante et/ou de la vaseline. Du fait de la combinaison d'une forte proportion de plastifiant huileux avec une relativement faible proportion d'élastomères tribloc, la matrice présente une très bonne compatibilité avec les tissus et de très bonnes propriétés de non adhérence à la plaie.

Parmi les plastifiants huileux convenant à la constitution de la matrice hydrophobe selon ce mode de réalisation, c'est-à-dire permettant la plastification de l'élastomère, on peut mentionner les huiles de paraffine, les huiles blanches médicales, les huiles minérales, la vaseline, les paraffine de baume, les huiles de silicone, les huiles végétales, les cires ou encore leurs mélanges.

De préférence, le plastifiant sera constitué d'un liquide ou d'un mélange de liquides compatible(s) avec la séquence centrale polyoléfine saturée des élastomères utilisés.

Parmi les composés plastifiants susceptibles d'être utilisés à cet effet, on peut citer en particulier les huiles minérales plastifiantes, quelle que soit la nature de la séquence centrale. On peut également citer les polybutènes - comme par exemple les produits commercialisés par la société BP CHEMICALS sous la dénomination NAPVIS® 10 ou encore des dérivés de phtalate tels que le dioctylphtalate ou le dioctyladipate, lorsque la séquence centrale est insaturée.

Alternativement, on peut aussi utiliser des produits de synthèse à base de mélanges liquides d'hydrocarbures saturés comme par exemple les produits commercialisés par la société TOTAL sous la dénomination GEMSEAL® et en particulier le produit GEMSEAL® 60 qui est un mélange isoparaffinique issu d'une coupe pétrolière totalement hydrogénée. On utilisera de préférence ces produits avec un copolymère tribloc comportant une séquence centrale saturée.

Dans le cadre de la présente invention, on utilisera de préférence des huiles plastifiantes et en particulier des huiles minérales formées de composés de nature paraffinique, naphténique ou de leurs mélanges dans des proportions variables.

Parmi les huiles plastifiantes convenant particulièrement, on peut citer les produits commercialisés par la société SHELL sous les dénominations ONDINA® et RISELLA® qui sont constitués de mélanges à base de composés naphténiques et paraffiniques ;

D'une façon particulièrement préférée, on utilisera une huile plastifiante minérale choisie parmi les produits commercialisés sous les dénominations ONDINA®963, ONDINA®917 et ONDINA®919.

Selon d'autres alternatives, il est possible d'autres huiles plastifiantes telles que l'huile Pionier® 2076P commercialisée par la société Hansen & Rosenthal, l'huile Puretol® 9D commercialisée par la société Petro Canada, et l'huile Marcol® 82 commercialisée par la société Exxon.

Selon un mode de réalisation préféré de l'invention, le plastifiant huileux, avantageusement l'huile plastifiante, est présent à hauteur de 20 à 85 % en poids par rapport au poids total de la matrice élastomérique, préférentiellement de 50 à 80 %, et de façon encore plus préférée d'environ 75%.

Selon un mode de réalisation particulier, une quantité d'huile choisie peut être substituée en totalité ou en partie à une quantité équivalente en vaseline Codex A® commercialisée par la société Aiglon®. D'une autre façon, une quantité de vaseline peut être simplement ajoutée à la quantité d'huile choisie au sein de la matrice élastomérique hydrophobe.

Selon un mode de réalisation préféré de la présente invention, la vaseline représente de 0 à 18% du poids total de la matrice élastomérique.

La matrice peut également contenir des antioxydants. Comme antioxydants adaptés, on peut citer les antioxydants phénoliques, par exemple les produits commercialisés sous la dénomination IRGANOX® 1010, IRGANOX® 565 et IRGANOX® 1035 par la société BASF.

Dans le cadre de la présente invention, le composé IRGANOX®1010 est préféré.

Selon un mode de réalisation particulier de l'invention, l'antioxydant est présent à hauteur de 0.05 à 5 %, préférentiellement de 0,1 à 3 % en poids par rapport au poids total de la matrice élastomérique

La matrice élastomérique hydrophobe selon un mode de réalisation particulier de l'invention peut comprendre en outre un additif choisi parmi le groupe constitué des agents stabilisateurs, des auxiliaires d'extrusion, des charges de remplissage, des pigments, des colorants, des agents de réticulation, des agents anti-odorant, des agents d'adhérence, des agents de compatibilité et leurs combinaisons.

La quantité d'additif incorporé dans la composition élastomère sera avantageusement de l'ordre de 0,1 à 7,5 % en poids, de préférence de 1 à 5 % en poids, rapportée au poids total de la matrice élastomérique.

En outre ladite matrice peut comprendre au moins un type de particules organiques ou inorganiques hydrophiles fixant l'eau tel que les hydrocolloïdes ou les polymères superabsorbants. Comme hydrocolloïdes appropriés, on peut citer par exemple la pectine, les alginates, les gommes végétales naturelles comme en particulier la gomme de Karaya, les dérivés de cellulose tels que les carboxyméthylcelluloses et leurs sels de métal alcalin tels que le sodium ou le calcium, ainsi que les polymères synthétiques à base de sels de l'acide acrylique, connus sous l'appellation "superabsorbants", comme par exemple les produits commercialisés par la société BASF sous la dénomination LUQUASORB® 1003 ou par la société CIBA Specialty Chemicals sous la dénomination SALCARE® SC91 ainsi que les mélanges de ces composés.

Les hydrocolloïdes préférés dans le cadre de la présente invention sont les sels de métal alcalin de la carboxyméthylcellulose, et en particulier la carboxyméthylcellulose de sodium (CMC) connue sous la référence CMC Blanose® 7H4XF. La taille des particules d'hydrocolloïdes est par exemple comprise entre 50 et 100 microns, notamment de l'ordre de 80 microns.

La quantité d'hydrocolloïdes incorporés dans la composition élastomère sera avantageusement de l'ordre de 2 à 20 % en poids, de préférence de 5 à 18 % en poids, de préférence encore de 8 à 18 % en poids, de préférence encore de 12 à 16 % en poids, rapportée au poids total de la matrice élastomérique.

Un agent actif peut également être rajouté au sein de cette matrice. Cet agent actif peut être un agent favorisant la cicatrisation ; un agent anti-viral, un agent antifongique, un agent anti-douleur, un agent anti-bactérien, un agent anti-inflammatoire, un agent kératolytique, un agent immunomodulateur, un agent immunomodulateur, un agent chélateur, un agent modifieur, un agent de la matrice extra-cellulaire.

Par " agent favorisant la cicatrisation ", on entend au sens de la présente invention, tout composé choisi parmi la liste non limitative constituée du Rétinol, de la Vitamine A, de la Vitamine E et ses dérivés, de la N-Acétyl Hydroxyproline, des extraits de Centella Asiatica, de la Papaïne, de l'acide Hyaluronique, des oligosaccharides polysulfatés synthétiques ayant 1 à 4 unités d'osés, leurs sels et complexes, tels que par exemple les sels alcalins de sucrose octasulfate (parmi lesquels on retrouve entre autres, le sel de potassium de sucrose octasulfate ou encore le sel de sodium de sucrose octasulfate), le sucralfate, les sels d'acidés aminés de sucrose octasulfate, ou encore le sel d'argent de sucrose octasulfate, de l'allantoïne, et de la metformine.

Par " agent anti-viral ", on entend au sens de la présente invention, tout composé choisi parmi la liste non limitative constituée de l'acyclovir, du famciclovir et du ritonavir.

Par " agent antifongique ", on entend au sens de la présente invention, tout composé choisi parmi la liste non limitative constituée de la nystatine, de l'amphotéricine B, de la natamycine, du miconazole, du kétoconazole, du clotrimazole, de l'éconazole, du bifonazole, du butoconazole, du fenticonazole, de l'isoconazole, de l'oxiconazole, du sertaconazole, du sulconazole, du thiabendazole, du tioconazole, du fluconazole, de l'itraconazole, du ravuconazole, du posaconazole, du voriconazole, de l'allylamine, de la terbinafine, de l'amorolfine, de la naftifine, et du butenafine.

Par "agent anti-douleur ", on entend au sens de la présente invention, tout composé choisi parmi la liste non limitative constituée du paracétamol, de la codéine, du dextropropoxyphène, du tramadol, de la morphine et ses dérivés et des corticoïdes et ses dérivés.

Par " agent anti-bactérien ", on entend au sens de la présente invention, tout composé choisi parmi la liste non limitative constituée des sels ou complexes d'argent (tels les sulfates d'argent, les nitrates d'argent, les sulfamides d'argent, la sulfadiazine argentique ou encore les zéolites à base d'argent), les sels de zinc ou de cuivre, le métronidazole, la néomycine, les pénicillines, l'acide clavulanique, les tétracyclines, la mynocycline, la chlorotétracycline, les aminoglycosides, l'amikacine, la gentamicine, ou encore les probiotiques.

Par " agent anti-inflammatoire ", on entend au sens de la présente invention, tout composé choisi parmi la liste non limitative constituée des antiinflammatoires non stéroïdiens (AINS), de l'aspirine ou acide acétylsalicylique, de l'ibuprofène, du kétoprofène, du flurbiprofène, du diclofenac, l'acéclophénac, du kétorolac, du méloxicam, du piroxicam, du ténoxicam, du naproxène, de l'indométacine, du naproxcinod, le nimésulid, du célécoxib, de l'étoricoxib, le parécoxib, le rofécoxib, du valdécoxib, de la phénylbutazone, de l'acide niflumique, et de l'acide méfénamique.

Par " agent kératolytique ", on entend au sens de la présente invention, tout composé choisi parmi la liste non limitative constituée de l'acide salicylique, du salicylate de zinc, de l'acide ascorbique, des acides alpha hydroxylés (tels que les acides glycolique, lactique, malique, citrique, tartrique), des extraits d'Erable argenté, de Griottier, ou de Tamarinier, de l'urée, de la Kératoline (commercialisée par la société Sederma), et de la papaïne.

Enfin, par « agent immunomodulateur », on entend le beta glucane, par « agent chélateur », on entend l'acide éthylène diamine tétraacétique (EDTA) ainsi que les acides hydroxamiques, par « agent modifieur » de pH, on entend le bicarbonate de sodium, et par « agent de la matrice extra-cellulaire », on entend, les glycosaminoglycanes, l'acide hyaluronique, le dermatane sulfate, l'héparane sulfate, les élastines, et la laminine.

De façon préférée un biguanide, tel que la metformine ou le polyhexaméthylène-biguanide (PHMB), ou encore le stéarate de PHMB est ajouté à ladite matrice.

Selon un mode de réalisation particulier de l'invention, l'agent actif est présent à hauteur de 1 à 7,5 % en poids par rapport au poids total de la matrice élastomérique, de préférence 5%.

En outre, la matrice élastomérique peut inclure si nécessaire un agent de relargage tel que le tensioactif MONTANOX® 80 qui est un polysorbate 80 ou le polymère SEPINOV® EMT 10 tous les deux commercialisés par la société SEPPIC pour optimiser la vitesse de gélification, la mouillabilité ou le relargage d'actifs éventuellement présents dans la composition.

Selon un mode de réalisation particulier de l'invention, l'agent de relargage est présent à hauteur de 1 à 15 % en poids par rapport au poids total de la matrice élastomérique.

Si l'on souhaite que la matrice élastomérique soit micro-adhérente, la matrice élastomérique hydrophobe peut contenir un produit tackifiant. Les produits tackifiants susceptibles d'être utilisés dans le cadre de la présente invention peuvent être choisis parmi les résines tackifiantes, les polyisobutylènes de bas poids moléculaire ou leurs mélanges.

Par matrice élastomérique hydrophobe micro-adhérente, on entend toute matrice pour pansement ayant un pouvoir adhésif sur plaque d'acier compris entre 0,5 et 100 cN/cm, de préférence entre 5 et 40 cN/cm. Ce pouvoir adhésif est mesuré selon la méthode EN 1939 dans laquelle un échantillon de pansement de 20 mm de large et 150 mm de long est posé sur une plaque d'acier et dans laquelle on mesure, au bout de 10 minutes, le pouvoir adhésif avec un dynamomètre à une vitesse de traction de 100 mm/min avec un angle de 90°.

Parmi les résines tackifiantes susceptibles d'être utilisées selon l'invention, on peut mentionner les résines polyterpènes ou terpènes modifiées, les résines de colophane, les résines hydrocarbonnées, les mélanges de résines cycliques, aromatiques et aliphatiques, ou des mélanges de ces résines.

De tels produits sont commercialisés par exemple :
- par la société ARAKAWA Chemical Industries sous la dénomination ARKON® P qui sont des résines polycyclopentadiènes hydrogénées ;
- par la société EXXON Chemical sous la dénomination ESCOREZ® et en particulier la série des résines 5000 qui sont hydrogénées.
- par la société GOODYEAR sous la dénomination WINGTACK®, et en particulier WINGTACK® 86 qui est une résine de synthèse formée de copolymères en C5/C9 ou WINGTACK® 10 qui est une résine à base de polyterpène synthétique ;
- par la société HERCULES sous la dénomination KRISTALEX® et en particulier KRISTALEX® 3085 qui est une résine à base d'alpha-méthylstyrène.
- par la société Kolon Industries sous la dénomination Sukorez® de grades : SU-90; SU-100; SU-100S

De façon générale, afin d'éviter les problèmes de coloration et de stabilité des résines insaturées, on préférera l'utilisation de résines hydrogénées, en particulier avec les copolymères triblocs à séquence centrale saturée car elles sont beaucoup plus compatibles avec ces derniers que les résines insaturées type WINGTACK que l'on utilise essentiellement avec les copolymères triblocs à séquence centrale insaturée.

Parmi ces dernières on préférera utiliser les résines ESCOREZ® de la série 5000 et tout particulièrement la résine ESCOREZ® 5380.

Les résines tackifiantes peuvent être utilisées seules ou en mélange avec d'autres produits tackifiants, de préférence dans une proportion de 5 à 50 % en poids, et plus particulièrement de 15 à 40 % en poids, rapportée au poids total de la composition.

Parmi les polyisobutylènes de bas poids moléculaire susceptibles d'être utilisés comme produits tackifiants on peut citer les polyisobutylènes ayant un poids moléculaire de l'ordre de 40 000 à 80 000 daltons, comme par exemple les produits commercialisés par la société BASF sous la dénomination OPPANOL® et en particulier les produits commercialisés sous les dénominations OPPANOL®B12 et OPPANOL®B15 ou par la société EXXON Chemical sous la dénomination Vistanex et en particulier le grade LM-MH.

Ces polyisobutylènes peuvent être utilisés seuls ou en mélange avec d'autres tackifiants en association avec les copolymères triblocs à séquence centrale insaturée. Leur proportion pourra varier dans ce cas entre 5 à 30 % en poids, et plus particulièrement de 8 à 15 % en poids, rapportée au poids total de la composition.

Il est possible de définir la matrice comme étant une matrice hydrophobe élastomérique non absorbante.

On entend par matrice élastomérique hydrophobe « non absorbante», toute matrice élastomérique hydrophobe telle que décrite précédemment et qui absorbe moins de 35% d'une solution saline (solution de NaCl à 0.8%) en 24h par rapport au poids sec de la matrice.

### Support

Le support selon la présente invention présente avantageusement des trous traversants.

D'une façon générale, ce support peut se présenter sous forme d'un tissu à mailles larges ouvertes et peut être obtenu par des procédés de tissage ou de tricotage permettant de former des mailles ouvertes de taille régulière, rectangulaires, carrées ou polygonales. Dans le cas d'un tissage, les mailles peuvent être fixées au moyen de fils de tour afin d'obtenir une bonne stabilité dimensionnelle. La dimension des mailles est telle que la surface unitaire des ouvertures est de l'ordre de 0,5 à 10 mm², préférentiellement 0,5 à 3 mm², le taux d'ouverture du tissu (rapport de la surface ouverte sur la surface totale) étant de l'ordre de 50 à 90 %. D'une façon générale, le fil utilisé pour fabriquer le tissu est constitué de filaments continus ou non continus, extensibles ou non extensibles et élastiques ou non élastiques. Ce fil est préférentiellement un fil continu à filaments, très peu extensible et non élastique, l'extensibilité ou l'allongement étant inférieure à 35 %. Par fil continu à filaments, on entend un fil formé de un ou plusieurs filaments longs retords ; le choix de filaments longs permet d'éviter les fibres courtes qui risquent de se détacher du support et venir se disperser près de la surface de contact avec la plaie.

Pour la même raison, le matériau constitutif des fils est de préférence de type hydrophobe, de nature artificielle ou synthétique ; ces constituants, comme par exemple les polyesters, les polyamides, les acétates de cellulose permettent d'obtenir des filaments longs et des fils présentant beaucoup moins de fibrilles que les fils obtenus à partir de fibres courtes par exemple. Le choix de certains matériaux synthétiques tels que des polyesters donne également la possibilité de thermofixer la structure à mailles larges du support. Le tissu à mailles larges est préférentiellement réalisé avec des fils de même nature, mais on peut utiliser aussi des tissus fabriqués par exemple avec des fils de chaîne et des fils de trame qui seraient de nature différente. Enfin, un autre avantage des matériaux très peu extensibles et non élastiques, tels que les polyesters, est une mise en oeuvre plus facile pendant le procédé de recouvrement des fils du tissu par la matrice élastomérique hydrophobe.

Selon un mode préféré de réalisation de l'invention, on applique la masse élastomérique hydrophobe sur le tissu à mailles larges de façon à enrober les fils du tissu en laissant une majorité des mailles non obstruée.

Comme indiqué précédemment, on utilise un support tissé ou tricoté présentant des mailles larges rectangulaires, carrées ou polygonales dont l'ouverture correspond sensiblement à 4 à 20 mailles par cm, le tissu ayant un taux d'ouverture (rapport des surfaces ouvertes sur la surface totale) de 50 à 90 %. Le fil utilisé pour obtenir le support est de préférence un fil continu à filaments, et on choisit, pour réaliser les exemples préférés de l'invention, des fils en matériau artificiel ou synthétique, à caractère hydrophobe et d'extensibilité inférieure à 35 %.

La nature du fil est, par exemple, un polyester de type polyéthylène téréphtalate, un polyamide ou un acétate de cellulose ; on utilise de préférence un tissu à mailles larges thermofixées en fils continus de polyester (Tergal ou polyéthylène téréphtalate), par exemple des tissus commercialisés sous le nom de marquisette, grammant environ 30 à 80 g/m². Ces tissus pratiquement non extensibles dans les directions chaîne ou trame présentent l'avantage de se travailler plus facilement que les tissus élastiques et on obtient un enrobage plus régulier des fils.

Ce type de support formé d'un tissu de fils en matériau flexible et très peu extensible et non élastique est notamment décrit dans la demande de brevet WO 00/16725.

Alternativement, le tissu constituant le support peut être un tricot thermofixé avec fils tramés, lesdits fils étant des fils continus à filaments non élastiques, qui présente dans le sens transversal une extensibilité mesurée selon la norme NF EN 13726-4 comprise entre 0,01 et 0,5 N/cm, de préférence comprise entre 0,05 à 0,3 N/cm, et de préférence encore entre 0,08 à 0,15 N/cm.

Selon une version préférée de la présente invention, ce tricot présentera dans le sens longitudinal une extensibilité mesurée selon la même norme comprise entre 15 et 30 N/cm et de préférence entre 20 et 25 N/cm. Une telle extensibilité dans le sens longitudinal permet en effet un enrobage plus facile et plus régulier du tricot par la matrice élastomérique hydrophobe du point de vue industriel.

De façon générale, ce tricot est réalisé avec fils tramés, et sera notamment fabriqué selon la technologie dite « mailles jetées ».

De plus, ce tricot est thermofixé. Cette thermofixation permet de stabiliser sur le plan dimensionnel la structure du tricot après tricotage par effet thermique. Cette opération de thermofixation est couramment utilisée par l'homme de l'art lors de la fabrication d'un tricot dont on veut figer la structure tridimensionnelle. Elle peut être mise en oeuvre à l'aide de différentes technologies soit par passage du tricot dans une série de fours thermorégulés, soit par passage du tricot dans un autoclave, soit encore par passage du tricot entre un ou plusieurs cylindres chauffés. Dans le cadre de la présente invention, on préférera réaliser cette opération de thermofixation par passage du tricot entre 2 cylindres chauffés.

Selon l'invention, ce tricot est réalisé à l'aide de fils continus à filaments non élastiques. Par fil continu à filaments, on entend un fil formé de un ou plusieurs filaments longs retords.

Ce fil sera de préférence choisi parmi les fils de 33 à 115 dtex comprenant 12 à 36 filaments.

Le matériau constitutif des fils est de préférence de nature synthétique et hydrophobe. Ce matériau est avantageusement choisi parmi les polyesters et les polyamides.

Selon la version préférée de l'invention, on utilisera un tricot avec fils tramés thermofixé à base de fils continus en polyester 50 dtex comprenant 24 filaments.

Ce tricot peut présenter un grammage compris entre 20 à 40 g/m² et de préférence entre 24 à 32 g/m².

Ce tricot peut présenter des mailles rectangulaires, carrées ou polygonales.

Ces mailles présenteront avantageusement des ouvertures dont la surface unitaire avant enrobage est de l'ordre de 0,5 à 3 mm² et de préférence comprise entre 0,85 et 1,25 mm².

Selon un mode préféré de réalisation, on utilisera un tricot qui présente des mailles trapézoïdales.

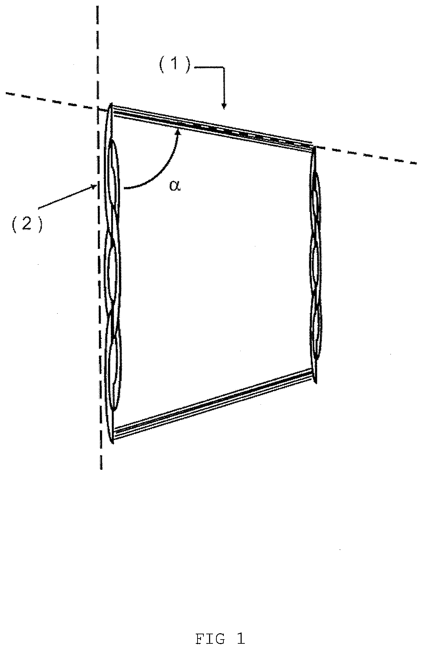

Selon une version préférée de la présente invention, on utilisera un tricot qui présente des mailles trapézoïdales. Un tricot avec une telle maille est illustré de façon schématique sur la figure 1. L'angle α est défini comme l'angle de raccordement moyen des rangées (1) et des colonnes (2) du tricot.

Selon la version préférée de la présente invention, ce tricot présente une surface de maille moyenne de l'ordre de 0,95 mm² et un angle α tel que décrit sur la figue 1 compris entre 75 et 85 degrés.

L'enduction de la matrice élastomérique hydrophobe à l'état fondu sur le tricot définit selon ce premier mode de réalisation est effectué de façon à laisser les mailles essentiellement non obturées, selon les techniques connues de l'homme de l'art et de manière à obtenir un grammage d'enduction qui varie de 100 à 160 g/m² et de préférence de 125 à 145 g/m².

Dans le cadre de la présente invention, pour réaliser cette enduction on préfèrera mettre en oeuvre le procédé décrit dans la demande de brevet WO 00/16725.

La figure 1 annexée représente de façon schématique une maille du tissu d'un pansement selon un mode de réalisation préféré de l'invention.

L'association d'une telle matrice élastomérique à un support tel que réalisé selon ce premier mode de réalisation permet d'obtenir un pansement dont les propriétés d'allongement à la rupture se trouvent sensiblement augmentées par rapport à un pansement ne comprenant qu'une seule de ces deux variantes, ou par rapport à tout autre pansement interface divulgué dans la littérature.

Selon un deuxième mode de réalisation alternatif, on peut utiliser un autre type de support formé d'un tissu à maille ouverte constituée par des fils peu extensibles et peu élastiques. Ces fils sont de préférence en polyester. Il s'agit de fils multi-filaments tissés de diamètre compris entre 10 et 20 µm et préférentiellement de 16 µm environ. L'ouverture des mailles est comprise entre 1 et 1.1 mm² et de préférence de 1.05 mm² environ. Ce support comprend de préférence un nombre de 7 fils par cm pris dans son sens longitudinal, et un nombre de 8 fils par cm pris dans son sens transversal. Le grammage de ce support est compris entre 40 et 45 g/cm² et est de préférence de 43 g/cm² environ. Enfin son extensibilité à 20% a été mesurée selon la norme NF EN 13726-4. Cette dernière est comprise entre 20 et 30 N/cm dans le sens longitudinal, et est préférentiellement de 24 N/cm. De la même manière, cette extensibilité dans le sens transversal est comprise entre 1 et 5 N/cm et est de préférence de 3 N/cm.

Selon un troisième mode de réalisation alternatif, on peut utiliser un support tissé ou tricoté présentant des mailles larges rectangulaires, carrées ou polygonales dont l'ouverture correspond sensiblement à 4 à 20 mailles par cm, le tissu ayant un taux d'ouverture (rapport des surfaces ouvertes sur la surface totale) de 50 à 90 %. Le fil utilisé pour obtenir le support est de préférence un fil continu à filaments, et on choisit des fils en matériau artificiel ou synthétique, à caractère hydrophobe et d'extensibilité inférieure à 35 %. La nature du fil est, par exemple, un polyester de type polyéthyltéréphtalate, un polyamide ou un acétate de cellulose ; on utilise de préférence un tissu à mailles larges thermofixées en fils continus de polyester (Tergal ou polyéthyltéréphtalate), par exemple des tissus commercialisés sous le nom de marquisette, grammant environ 30 à 80 g/m². Ces tissus pratiquement non extensibles dans les directions chaîne ou trame présentent l'avantage de se travailler plus facilement que les tissus élastiques et on obtient un enrobage plus régulier des fils.

### Pansement

Selon un mode de réalisation préféré de la présente invention, le support textile est enrobé par la matrice élastomérique hydrophobe.

Cet enrobage se fait par passage dans un bain de matrice élastomérique qui se trouve à l'état fondu à 135-145°C, puis l'excédent est éliminé par passage entre deux cylindres fixes dont l'écart est prédéterminé en fonction du grammage d'enduction recherché. Le support est ensuite refroidi par un courant d'air froid ascendant. La quantité de matrice élastomérique à l'état fondu déposée sur les fils du tissu est comprise entre 50 et 300 g/m2, de préférence entre 100 et 150 g/m2. Le grammage du pansement global (support et matrice élastomérique) est compris entre 90 et 1700 g/m², et de préférence compris entre 100 et 800 g/m².

Selon un mode de réalisation tout à fait particulier de la présente invention, ce pansement ou cette matrice élastomérique peuvent servir d'enveloppe pour un matériau de remplissage de plaie cavitaire en thérapie par pression négative tel que décrit dans les demandes de brevet WO2009/071928 et WO2009/071938 de Smith and Nephew ou dans la demande des Laboratoires Urgo déposée sous le numéro FR 13 63162.

### Exemples

La présente invention est illustrée plus en détails dans les exemples non limitatifs suivants.

### Exemple 1 : Réalisation d'un pansement selon l'objet de l'invention

On prépare la matrice élastomérique à l'aide d'un mélangeur vertical à environ 150°C par mélange de 15.2 kg d'huile de paraffine (ONDINA 919 commercialisée par la société SHELL) soit au total 75 % environ de la masse de la matrice), et 3,04 kg (soit 15 % environ de la masse totale de la matrice élastomérique finale) de carboxyméthylcellulose sodique (ref. Blanose 7H4XF commercialisée par Ashland). Lorsque la carboxyméthylcellulose sodique est dispersée dans l'huile, on ajoute 1,2 kg (soit environ 5 % de la masse de la matrice) de vaseline (qualité conforme à la pharmacopée française ou Codex). Après fusion de la vaseline, on ajoute 1 kg (soit environ 5 % de masse totale de la matrice élastomérique finale) d'un mélange d'élastomères de type Septon 4055 et Septon 4077 (dans une proportion de 50% et 50% respective par rapport au pourcentage total d'élastomère introduit dans le mélange) et 25 g d'antioxydant (IRGANOX 1010). Après 45 à 60 minutes de mélange, la matrice élastomérique qui est à l'état fondu peut être utilisée pour enrober les fils du tissu. Le tissu utilisé est une marquisette thermofixée en fils de polyester (polyéthyltéréphtalate), 33 décitex en chaîne et en trame, présentant des mailles carrées dont l'ouverture est environ 0,8 à 1 mm² ; le grammage du tissu est environ 45 g/m² (ce tissu est fabriqué par la société TEXINOV). Le tissu est enrobé d'une couche de matrice élastomérique par passage dans un bain de matrice élastomérique à l'état fondu à 135-145°C, puis l'excédent est éliminé par passage entre deux cylindres fixes dont l'écart est prédéterminé en fonction du grammage d'enduction recherché. Le support textile enrobé est ensuite refroidie par un courant d'air froid ascendant. La quantité de matrice élastomérique déposée sur les fils du tissu est environ de 130 g/m². Le support textile enrobé est complexé avec un film protecteur en polyester d'épaisseur 23 µm sur chacune de ses faces, puis découpée en feuilles pour former des compresses, chacune étant conditionnée dans une pochette étanche et stérilisée sous rayonnement β.

### Exemple 2 : Mesures comparatives des pertes d'huiles de différentes matrices élastomériques pour pansements

La méthode de mesure consiste en plusieurs étapes, à savoir :
- la pesée avant la pose de chaque échantillon de produit testé (de dimension 4 X 4 cm et d'épaisseur 4 mm) sur un papier absorbant, type papier buvard, dudit papier absorbant en question.
- la pose de chaque échantillon de produit testé à l'étape précédente sur un papier absorbant pendant 1 heure ou 4 heures à 37°C.
- La pesée de chaque papier absorbant postérieurement à sa mise en contact avec l'échantillon de produit testé.

Les échantillons testés sont :
- La matrice élastomérique pour pansement telle que décrite dans l'exemple 2 du brevet EP 2 524 706. Cet exemple divulgue une matrice élastomérique pour pansement comprenant une association d'un Septon 4055 et 4077, la part en élastomères étant de 2,6% par rapport au poids total de la matrice.
- La matrice élastomérique pour pansement telle que décrite dans l'exemple 7 du brevet EP 1 143 895. Cet exemple divulgue la présence d'un seul élastomère de type Septon 4055 à hauteur de 5% par rapport au poids total de ladite matrice.
- Le pansement selon l'exemple 1 de la présente invention.

Les résultats des mesures des pertes d'huiles des différents échantillons testés (n=6) sont donnés au sein du tableau suivant :

| Temps de pose du produit testé sur le papier absorbant | Matrice élastomérique du produit selon l'exemple 2 du brevet EP 2 524 706 | Matrice élastomérique du produit selon l'exemple 7 du brevet EP 1 143 895 | Matrice élastomérique du produit selon l'exemple 1 de la présente invention |
|---|---|---|---|
| 1 heure | 22 | 14 | Inférieur ou égal à 13 |
| 4 heures | 32 | 26 | Inférieur ou égal à 22 |

Les valeurs données sont exprimées en g/m².

On constate que la matrice entrant dans la composition du produit selon l'exemple 1 de la présente invention possède des niveaux de pertes d'huiles sensiblement inférieures à celles entrant dans la composition des produits comparatifs concernés. Ceci est dû à un effet surprenant et synergique du pourcentage d'élastomères introduit dans la matrice élastomérique et au choix des élastomères incorporés dans ladite matrice.

Les tendances exhaustives des résultats présentés dans ce tableau sont confirmées par des tests réalisés sur des durées plus longues, les différences exprimées en g/m² de quantité d'huile libérée étant significativement plus importantes encore.

Ainsi l'objet de la présente invention améliore la stabilité au conditionnement ou à l'usage d'un tel type de pansement.

## Revendications

1. Pansement comprenant un support et une matrice élastomérique hydrophobe enrobant ledit support, ladite matrice comprenant au moins deux élastomères de type SEPS, SEBS ou SEEPS, constitués d'une association de blocs polystyrène et de blocs polyoléfine, l'un de ces élastomères ayant un poids moléculaire compris entre 200000 et 350000 Dalton et l'autre de ces élastomères ayant un poids moléculaire compris entre 320000 et 600000 Dalton, ces poids moléculaires étant mesurés par chromatographie par perméation de gel, la part totale d'élastomères étant comprise entre 3,2 et 10% du poids total de ladite matrice élastomérique.

2. Pansement selon la revendication 1, **caractérisé en ce que** dans ladite matrice, l'un des élastomères a un poids moléculaire compris entre 240000 et 310000 Dalton et l'autre des élastomères a un poids moléculaire compris entre 320000 et 400000 Dalton, ces poids moléculaires étant mesurés par chromatographie par perméation de gel

3. Pansement selon la revendication 1 ou 2, **caractérisé en ce que** la matrice élastomérique comprend au moins une huile plastifiante naphténique et/ou paraffinique.

4. Pansement selon la revendication 3, **caractérisé en ce que** l'huile plastifiante représente de 20 à 85%, de préférence 75%, du poids total de ladite matrice élastomérique.

5. Pansement selon l'une quelconque des revendications précédentes, **caractérisée en ce que** la matrice élastomérique comprend de la vaseline.

6. Pansement selon la revendication précédente **caractérisé en ce que** la vaseline représente de 0 à 18% du poids total de la matrice élastomérique.

7. Pansement selon l'une quelconque des revendications précédentes, **caractérisé en ce que** la matrice élastomérique comprend au moins un hydrocolloide représentant de 2 à 20% du poids total de la matrice élastomérique.

8. Pansement selon l'une quelconque des revendications précédentes, **caractérisé en ce que** la matrice élastomérique comprend en outre un agent actif.

9. Pansement selon la revendication précédente **caractérisé en ce que** l'agent actif est le stéarate de polyhexaméthylène-biguanide hydrophobe, ledit agent représentant de 1 à 7.5% du poids total de la matrice élastomérique.

10. Pansement selon l'une quelconque des revendications précédentes, **caractérisé en ce que** le support est un textile formé par un assemblage de fils constitué de filaments continus ou non continus, lesdits filaments étant extensibles ou non extensible et élastiques ou non élastiques.

11. Pansement selon la revendication 10, **caractérisé en ce que** le support est un tissé formé de fils constitués de filaments continus extensibles et non élastiques.

## Patentansprüche

1. Wundverband, umfassend einen Träger und eine hydrophobe elastomere Matrix, die den Träger beschichtet, wobei die Matrix mindestens zwei Elastomere vom Typ SEPS, SEBS oder SEEPS umfasst, die aus einer Verbindung von Polystyrolblöcken und Polyolefinblöcken gebildet sind, wobei eines dieser Elastomere ein Molekulargewicht aufweist, das zwischen 200.000 und 350.000 Dalton beträgt, und das andere dieser Elastomere ein Molekulargewicht aufweist, das zwischen 320.000 und 600.000 Dalton beträgt, wobei diese Molekulargewichte durch Gelpermeationschromatographie gemessen werden, wobei der Gesamtanteil von Elastomeren zwischen 3,2 und 10 % des Gesamtgewichts der elastomeren Matrix beträgt.

2. Wundverband gemäß Anspruch 1, **dadurch gekennzeichnet, dass** in der Matrix eines der Elastomere ein Molekulargewicht aufweist, das zwischen 240.000 und 310.000 Dalton beträgt, und das andere der Elastomere ein Molekulargewicht aufweist, das zwischen 320.000 und 400.000 Dalton beträgt, wobei diese Molekulargewichte durch Gelpermeationschromatographie gemessen werden.

3. Wundverband gemäß Anspruch 1 oder 2, **dadurch gekennzeichnet, dass** die elastomere Matrix mindestens ein naphthenisches und/oder paraffinisches Weichmachendes Öl umfasst.

4. Wundverband gemäß Anspruch 3, **dadurch gekennzeichnet, dass** das Weichmachendes Öl 20 bis 85 %, vorzugsweise 75 % des Gesamtgewichts der elastomeren Matrix darstellt.

5. Wundverband gemäß einem der vorhergehenden Ansprüche, **dadurch gekennzeichnet, dass** die elastomere Matrix Vaseline umfasst.

6. Wundverband gemäß einem der vorhergehenden Ansprüche, **dadurch gekennzeichnet, dass** die Vaseline 0 bis vorzugsweise 18 % des Gesamtgewichts der elastomeren Matrix darstellt.

7. Wundverband gemäß einem der vorhergehenden Ansprüche, **dadurch gekennzeichnet, dass** die elastomere Matrix ein Hydrokolloid umfasst, das 2 bis vorzugsweise 20 % des Gesamtgewichts der elastomeren Matrix darstellt.

8. Wundverband gemäß einem der vorhergehenden Ansprüche, **dadurch gekennzeichnet, dass** die elastomere Matrix ferner einen Wirkstoff umfasst.

9. Wundverband gemäß einem der vorhergehenden Ansprüche, **dadurch gekennzeichnet, dass** der Wirkstoff hydrophobes Polyhexamethylenbiguanidstearat ist, wobei der Wirkstoff 1 bis 7,5 % des Gesamtgewichts der elastomeren Matrix darstellt.

10. Wundverband gemäß einem der vorhergehenden Ansprüche, **dadurch gekennzeichnet, dass** der Träger eine Textilie ist, die durch eine Anordnung von Fäden gebildet ist, die aus kontinuierlichen oder nicht-kontinuierlichen Fasern bestehen, wobei die Fasern dehnbar oder nicht dehnbar und elastisch oder nicht elastisch sind.

11. Wundverband gemäß Anspruch 10, **dadurch gekennzeichnet, dass** der Träger ein Gewebe ist, das aus Fäden gebildet ist, die aus dehnbaren und nicht elastischen kontinuierlichen Fasern bestehen.

## Claims

1. A wound dressing comprising a backing and a hydrophobic elastomeric matrix which coats said backing, said matrix comprising at least two elastomers of the type SEPS, SEBS or SEEPS, formed from polystyrene blocks combined with polyolefin blocks, one of these elastomers having a molecular weight comprised between 200000 and 350000 Daltons and the other of these elastomers having a molecular weight comprised between 320000 and 600000 Daltons, these molecular weights being measured by gel permeation chromatography, the total amount of elastomers being comprised between 3.2 and 10% of the total weight of the elastomeric matrix.

2. The dressing according to claim 1, **characterised in that** in said matrix, one of the elastomers has a molecular weight comprised between 240000 and 310000 Daltons and the other of these elastomers has a molecular weight comprised between 320000 and 400000 Dalton, these molecular weights being measured by gel permeation chromatography.

3. The dressing according to claim 1 or 2, **characterised in that** the elastomeric matrix comprises at least one naphthenic and/or paraffinic plasticizing oil.

4. The dressing according to claim 3, **characterised in that** the plasticizing oil represents from 20 to 85%, preferably 75%, of the total weight of said elastomeric matrix.

5. The dressing according to anyone of the preceding claims, **characterised in that** the elastomeric matrix comprises petroleum jelly.

6. The dressing according to the preceding claim, **characterised in that** the petroleum jelly represents from 0 to 18% of the total weight of the elastomeric matrix.

7. The dressing according to anyone of the preceding claims, **characterised in that** said elastomeric matrix comprises at least one hydrocolloid representing from 2 to 20% of the total weight of the elastomeric matrix.

8. The dressing according to anyone of the preceding claims, **characterised in that** the elastomeric matrix further comprises an active substance.

9. The dressing according to the preceding claim, **characterised in that** said active substance is the hydrophobic polyhexamethylene-biguanide stearate, said substance representing from 1 to 7.5% of the total weight of the elastomeric matrix.

10. The dressing according to anyone of the preceding claims, **characterised in that** the backing is a fabric formed from an assembly of yarns in the form of continuous or non-continuous filaments, said filaments being extensible or non-extensible and elastic or non-elastic.

11. The dressing according to claim 10, **characterised in that** said backing is a woven material formed from yarns in the form of non-elastic and extensible continuous filaments.
